# EUROPÄISCHE PATENTSCHRIFT

(11) **EP 3 229 918 B1**
(45) Veröffentlichungstag und Bekanntmachung des Hinweises auf die Patenterteilung: **13.03.2024**
(21) Anmeldenummer: 15794191.5
(22) Anmeldetag: 13.11.2015
(51) Int. Cl.: A61K 8/34, A61Q 5/06, B01D 3/06

(54) **ZUSAMMENSETZUNG UND VERFAHREN ZUR BEHANDLUNG KERATINISCHER FASERN MIT ENTSPANNUNGSVERDAMPFUNG**
COMPOSITION AND PROCEDURE FOR TREATING KERATINIC FIBERS WITH FLASH EVAPORATION
COMPOSITION ET PROCÉDURE POUR TRAITER LES FILAMENTS KERATINIQUES AVEC L'EVAPORATION INSTANTANÉE

(30) Priorität: 11.12.2014 DE 102014225554
(43) Veröffentlichungstag der Anmeldung: 18.10.2017
(73) Patentinhaber: Henkel AG & Co. KGaA, 40589 Düsseldorf (DE)
(72) Erfinder: MÜLLER, Burkhard, 40221 Düsseldorf (DE); KNAPPE, Thorsten, 22869 Schenefeld (DE)
(86) Internationale Anmeldenummer: PCT/EP2015/076538
(87) Internationale Veröffentlichungsnummer: WO 2016/091532

(56) Entgegenhaltungen:
- GB-A- 2 207 443
- US-A- 3 167 478
- US-A- 5 723 433

## Beschreibung

Die Anmeldung betrifft das technische Fachgebiet der Färbung keratinhaltiger Fasern, insbesondere menschlicher Haare. Gegenstand der Anmeldung sind spezifische haarkosmetische Formulierungen, welche sich zur Applikation auf keratinhaltige Fasern mittels eines Entspannungsverdampfungsverfahrens eignen. Darüber hinaus sind die Verwendung dieser haarkosmetischen Formulierungen in Vorrichtungen zur Entspannungsverdampfung und Verfahren zur temporären Umformung keratinhaltiger Fasern Gegenstand der vorliegenden Anmeldung.

Eine ansprechend aussehende Frisur wird heute allgemein als unverzichtbarer Teil eines gepflegten Äußeren angesehen. Um eine solche ansprechende Frisur zu realisieren werden die Haare kosmetischen Behandlungsverfahren unterworfen, die von der Reinigung mittels eines Shampoos bis zur permanenten Verformung mittels chemisch/thermischer Verfahren oder der permanenten oxidativen Farbaufhellung reichen. Zur Färbung der Haare stehen zeitlich begrenzt haltbare direktziehende Farbstoffe und Oxidationsfarbstoffe zur Verfügung. Diese Haarfarbstoffe werden in der Regel in Form flüssiger oder schaumförmiger Zubereitungen auf das Haar aufgebracht. Als Hilfsmittel bei der Applikation der Farbstoffzubereitungen werden beispielsweise Pinsel oder Schaumdispenser eingesetzt.

Zu den Schaumdispensern zählen insbesondere die Pumpsprays oder Aerosolsprays, mit deren Hilfe die kosmetischen Zubereitungen entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation haarkosmetischer Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann.

Vor diesem Hintergrund besteht ein Bedarf nach alternativen Wegen zur Zerstäubung oder Aufschäumung haarkosmetischer Zubereitungen. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation haarkosmetischer Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel.

Aufgabe der vorliegenden Erfindung war es daher, spezifische haarkosmetische Zubereitungen zur Färbung keratinhaltiger Fasern zur Verfügung zu stellen, welche sich aufgrund ihrer chemischen und physikalischen Eigenschaften zur zielgerichteten Sprühapplikation mittels einer Vorrichtung zur Entspannungsverdampfung eignen. Weiterhin sollen die Zubereitungen geeignet sein, nach einer Applikation mittels eines Entspannungsverfahrens eine gute kosmetische Wirkung zu erzielen. Es hat sich gezeigt, dass zur Lösung dieser Aufgabe aus der Vielzahl der bekannten haarkosmetisch wirksamen Zubereitungen insbesondere lösungsmittelhaltige Zubereitungen mit spezifischen Gewichtsanteilen an direktziehenden Farbstoffen geeignet sind.

Ein erster Gegenstand der vorliegenden Erfindung ist somit ein kosmetisches Produkt, umfassend
a) eine kosmetische Zubereitung, enthaltend bezogen auf ihr Gesamtgewicht
   a1) 65 bis 75 Gew.-% polares Lösungsmittel;
   a2) 0,003 bis 3,0 Gew.-% direktziehenden Farbstoff;
b) eine Vorrichtung zur Entspannungsverdampfung der kosmetischen Zubereitung a).

Die erfindungsgemäße kosmetische Zubereitung enthält als ersten wesentlichen Bestandteil 65 bis 75 Gew.-% mindestens eines polaren Lösungsmittels a1). Entsprechende Mittel zeichnen sich durch eine gute kosmetische Wirkung bei gleichzeitig guter Applizierbarkeit aus.

Ein zweiter wesentlicher Bestandteil erfindungsgemäßer kosmetischer Zusammensetzungen ist der direktziehende Farbstoff a2). Für die Herstellung und Lagerung wie für die kosmetischen Eigenschaften der kosmetischen Zubereitung a) hat es sich als vorteilhaft erwiesen, wenn der Gewichtsanteil des direktziehenden Farbstoffs a2) am Gesamtgewicht der kosmetischen Zubereitung a) 0,003 bis 3,0 Gew.-% beträgt.

Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen oder den Indophenolen und deren physiologisch verträglichen Salze.

Die erfindungsgemäßen kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur T₁ erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck p₀ < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, p₀ = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck p₀, zum Beispiel den umgebenden Luftdruck (p₀ = 1,000 bar), entlastet werden kann, wodurch mindestens teilweise eine Verdampfung der kosmetischen Zubereitung a) erreicht wird.

Die kosmetische Zubereitung a) kann unmittelbar in dem Raum entspannt werden, in welchem sie zuvor erhitzt wurde. Die erhitzte und unter Überdruck befindliche kosmetische Zubereitung a) kann alternativ aber auch nach dem Erhitzen in einen zweiten Raum transportiert werden, in welchem dann nachfolgend die Druckentlastung erfolgt.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung kann die kosmetische Zubereitung a) einer Düse zugeführt werden, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher bevorzugt. Der spezifische Düsentyp oder die spezifische Düsengestaltung wird in Abhängigkeit von den jeweiligen Sprühnebeleigenschaften gezielt festgelegt.

Zusammenfassend verfügt eine bevorzugte Vorrichtung zur Entspannungsverdampfung über
- einen mittels eines Ventils zu verschließenden und zu öffnenden Behälter b1), welcher den geschlossenen Innenraum definiert, in dem die kosmetische Zubereitung aufgenommen werden kann,
- eine Heizvorrichtung b3), welche es ermöglicht eine in dem Behälter b1) befindliche kosmetische Zubereitung zu erhitzen.

Besonders bevorzugt ist der Einsatz einer zusätzlichen Düse b4), welche eine Zerstäubung der aus dem Behälter entweichenden kosmetischen Zubereitung a) ermöglicht. Alternativ zu einem Ventil kann auch ein vergleichbar wirkendes Schließelement, welches eine zugehörige Öffnung im Behälter durch entsprechende Positionsänderung verschließen oder freigeben kann, zum Einsatz kommen.

Die kosmetische Zubereitung a) ist vorzugsweise flüssig. Die kosmetische Zubereitung a) kann als Lösung oder Dispersion, beispielsweise als Emulsion oder Suspension vorliegen. Bevorzugte kosmetische Zubereitungen a) liegen in Form einer Lösung oder einer Suspension vor.

Zur Verbesserung der Anwendungseigenschaften erfindungsgemäßer kosmetischer Zubereitungen bei gleichzeitiger Minimierung der thermischen Belastung etwaiger Wirk- oder Hilfsstoffe im Verlauf des Entspannungsverdampfungsverfahrens, hat es sich als vorteilhaft erwiesen, polare Lösungsmittel a1) einzusetzen, welche eine Siedetemperatur (20°C, 1013 mbar) zwischen 50 und 110°C, vorzugsweise zwischen 70 und 105°C aufweisen. Als besonders geeignet haben sich dabei Ethanol, Isopropanol und Wasser erwiesen, welche aus diesem Grund als polare Lösungsmittel a1) bevorzugt werden.

Besonders bevorzugte polare Lösungsmittel a1) oder Lösungsmittelsysteme sind dadurch gekennzeichnet, dass der Gewichtsanteil von Wasser am Gesamtgewicht des polaren Lösungsmittels a1) mehr als 80 Gew.-%, vorzugsweise mehr als 85 Gew.-% und insbesondere mehr als 90 Gew.-% beträgt.

In einer bevorzugten Ausführungsform wird als direktziehender Farbstoff a2) ein anionischer Farbstoff eingesetzt. Als anionische direktziehende Farbstoffe eignen sich insbesondere 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 5-Hydroxy-1-(4-sulfophenyl)-4-[(4-sulfophenyl)azo]pyrazol-3-carbonsäure-trinatriumsalz (C.I. 19,140; Food Yellow No. 4; Acid Yellow 23), 3-[(4-Phenylamino)phenyl]azobezolsulfonsäuresäure-natriumsalz (C.I. 13,065; Ki406; Acid Yellow 36), 4-[(2-Hydroxynaphth-1-yl)azo]-benzolsulfonsäure-natriumsalz (C.I. 15,510; Acid Orange 7), 6- Hydroxy-5-[(4-sulfonaphth-1-yl)azo]-2,4-naphthalin-disulfonsäure-trinatriumsalz (C.I. 16,255; Ponceau 4R; Acid Red 18), 8-Amino-1-hydroxy-2-(phenylazo)-3,6-naphthalin-disulfonsäure-dinatriumsalz (C.I. 17,200; Acid Red 33; Red 33), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 2',4',5',7'-Tetrabrom-4,5,6,7-tetrachlor-3',6'- dihydroxyspiro[isobenzofuran-1 (3H),9'[9H]xanthen]-3-on-dinatriumsalz (C.I. 45,410; Acid Red 92), 3-Hydroxy-4-[(4-methyl-2-sulfonphenyl)azo]-2-naphthalincarbonsäure-calciumsalz (C.I. 15,850:1; Pigment Red 57:1), 1,4-Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(cyclohexylamino)-9,10-anthrachinon-2-sulfonsäure-natriumsalz (C.I. 62045; Acid Blue 62), 1-Hydroxy-4-[(4-methyl-2- sulfophenyl)amino]-9,10-anthrachinon-natriumsalz (C.I. 60,730; D&C Violett No. 2; Acid Violet 43), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 3',3",4,5,5',5",6,7-Octabromphenolsulfonphthalein (Tetrabromphenolblau).

Bevorzugte anionische direktziehende Farbstoffe sind die unter den internationalen Bezeichnungen bzw. Handelsnamen Acid Yellow 1, Acid Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

In einer alternativen Ausführungsform wird als als direktziehender Farbstoff a2) ein kationischer Farbstoff eingesetzt.Als kationische direktziehende Farbstoffe eignen sich insbesondere Di[4-(diethylamino)phenyl][4-(ethylamino)naphthyl]carbenium-chlorid (C.I. 42,595; Basic Blue 7), Di[4-(dimethylamino)phenyl][4-(phenylamino)naphthyl] carbenium-chlorid (C.I. 44,045; Basic Blue 26), 8-Amino-2-brom-5-hydroxy-4-imino-6-[(3-(trimethylammonio)phenyl)amino]-1(4H)-naphthalinon-chlorid (C.I. 56,059; Basic Blue No. 99), Tri(4-amino-3-methylphenyl)carbenium-chlorid (C.I. 42,520; Basic Violet 2), Di(4-aminophenyl)(4-amino-3- methylphenyl)carbeniumchlorid (C.I. 42,510 Basic Violet 14), 1-[(4-Aminophenyl)azo]-7-(trimethylammonio)- 2-naphthol-chlorid (C.I. 12,250; Basic Brown 16), 1-[(4-Amino-3-nitrophenyl)azo]-7-(trimethylammonio)-2-naphthol-chlorid (C.I. 12,251; Basic Brown 17), 3-[(4-Amino-2,5- dimethoxyphenyl)azo]-N,N,N-trimethylbenzolaminium-chlorid (C.I. 12,605, Basic Orange 69), 2-[((4-Dimethylamino)phenyl)azo]-1 ,3-dimethyl-1H-imidazoliumchlorid (Basic Red 51), 2-Hydroxy-1-[(2-methoxyphenyl)azo]-7-(trimethylammonio)-naphthalin-chlorid (C.I. 12,245; Basic Red 76), 2-[4-Aminophenyl]azo]-1,3-dimethyl-1H-Imidazolium-chlorid (Basic Orange 31), 3-Methyl-1-phenyl-4-[(3-(trimethylammonio)phenyl)azo]-pyrazol-5-on-chlorid (C.I. 12,719; Basic Yellow 57), 1-Methyl-4-((methylphenylhydrazono)methyl)-pyridinium-methylsulfat (Basic Yellow 87), 1-(2- Morpholiniumpropylamino)-4-hydroxy-9,10-anthrachinon-methylsulfat, 4-Formyl-1-methylquinolonium-p-toluensulfonat und direktziehende Farbstoffe, die einen Heterocyclus enthalten, der mindestens ein quaternäres Stickstoffatom aufweist.

In einer weiteren Ausführungsform wird als als direktziehender Farbstoff a2) ein nichtionischer Farbstoff eingesetzt. Als nichtionische direktziehende Farbstoffe eignen sich insbesondere nichtionische Nitro- und Chinonfarbstoffe und neutrale Azofarbstoffe.

Geeignete blaue Nitrofarbstoffe sind insbesondere 1,4-Bis[(2-hydroxyethyl)amino]-2-nitrobenzol (HC Violet BS), 1-(2-Hydroxyethyl)amino-2-nitro-4-[di(2-hydroxyethyl)amino]-benzol (HC Blue 2), 4-[Di(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol-hydrochlorid (HC Blue 12), 1-(2-Hydroxyethyl)amino-2-nitro-4-N-ethyl-N-(2-hydroxyethyl)aminobenzol (HC Blue 15), 1-Amino-3-methyl-4-[(2-hydroxyethyl)amino]-6-nitrobenzol (HC Violet 1), 1-(3-Hydroxypropylamino)-4-[di(2-hydroxyethyl)amino]-2-nitrobenzol (HC Violet 2).

Geeignete rote Nitrofarbstoffe sind insbesondere 1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1), 1-Amino-4-[di(2-hydroxyethyl)amino]-2-nitrobenzol-hydrochlorid (HC Red 13), 1-Amino-4-[(2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)-amino]-3-nitrotoluol, 4-Amino-3-nitrophenol, 4-[(2-Hydroxyethyl)-amino]-3-nitrophenol, 4-[(2-Nitrophenyl)amino]phenol (HC Orange 1), 1-[(2-Aminoethyl)amino]-4-(2-hydroxyethoxy)-2-nitrobenzol (HC Orange 2), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2- Hydroxyethyl)amino]-4,6-dinitrophenol und deren Salze, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 1,2,3,4-Tetrahydro-6-nitrochinoxalin, 6-Hydroxy-5-((2-methoxy-5-methyl-4-sulfophenyl)azo)- 2-naphthalensulfonsäure (Curry Red).

Geeignete gelbe Nitrofarbstoffe sind insbesondere 1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)-amino]-3-nitro-1-trifluormethyl-benzol (HC Yellow 6), 2-[(2- Hydroxyethyl)amino]-1-methoxy-5-nitrobenzol, 2-Amino-4-nitrophenol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluormethyl-benzol (HC Yellow 13).

Geeignete Chinonfarbstoffe sind insbesondere 1-[(2-Hydroxyethyl)amino]-4-methylamino-9,10-anthrachinon (C.I. 61,505, Disperse Blue 3), Mischungen aus 1,4-bis[(2-hydroxyethyl)amino]anthra-9,10-quinon mit 1-[(2-hydroxyethyl)amino]-4-[(3-hydroxypropyl)amino]anthra-9,10-quinon und 1,4-bis[(3-hydroxypropyl)amino]anthra-9,10-quinone (Disperse Blue 377), 1,4-Diamino-9,10-anthrachinon (C.I. 61,100, Disperse Violet 1), 1-Amino-4-(methylamino)-9,10-anthrachinon (C.I. 61,105, Disperse Violet 4, Solvent Violet No. 12), 2-Hydroxy-1,4-naphthochinon (Lawsone, C.I. 75,480, Natural Orange 6), 1,4-bis[(2,3-dihydroxypropyl)amino]-9,10-anthracenedion (HC Blue 14).

Geeignete neutrale Azofarbstoffe sind insbesondere 1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4- Aminophenyl)azo]-1-[di(2-hydroxyethyl)amino]-3-methylbenzol (HC Yellow 7), 2,6-Diamino-3-[(pyridin-3-yl)azo]-pyridin, 4-[(4-Nitrophenyl)azo]-anilin (C.I. 11,005; Disperse Orange 3).

Ein bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 65 bis 75 Gew.-% polares Lösungsmittel;
   a2) 0,003 bis 3,0 Gew.-% direktziehenden Farbstoff;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht ein in dem Behälter b1) befindliche kosmetische Zubereitung zu erhitzen
   - eine Düse b3), welche eine Zerstäubung der kosmetischen Zubereitung a) ermöglicht.

Ein besonders bevorzugter Gegenstand der vorliegenden Erfindung ist mit anderen Worten ein kosmetisches Produkt, umfassend
a) eine kosmetische Zubereitung, enthaltend bezogen auf ihr Gesamtgewicht
   a1) 65 bis 75 Gew.-% polares Lösungsmittel;
   a2) 0,003 bis 3,0 Gew.-% direktziehenden Farbstoff;
   b) eine Vorrichtung zur Entspannungsverdampfung der kosmetischen Zubereitung a), wobei die Vorrichtung zur Entspannungsverdampfung einen Behälter b1) und eine Heizvorrichtung b3 umfasst und derart ausgestaltet ist, dass
      - die kosmetische Zubereitung a) in den Innenraum des Behälters b1) aufgenommen werden kann,
      - der mit der kosmetischen Zubereitung a) wenigstens anteilsweise befüllte Innenraum des Behälters b1) verschlossen werden kann,
      - die kosmetische Zubereitung a) in dem geschlossenen Innenraum des Behälters b1) mittels der Heizvorrichtung b3) unter Druckerhöhung erhitzt werden kann,
      - die erhitzte kosmetische Zubereitung a) aus dem Innenraum des Behälters b1) unter Druckminderung in die Umgebung entspannt werden kann.

Der Behälter b1), in welchem die kosmetische Zubereitung erhitzt wird, ist dabei in einer Weise ausgestaltet, die es ermöglicht, diesen Behälter während der Erhitzung der kosmetischen Zubereitung a) gegen die Umgebung vollständig abzuschließen und nach der Erhitzung, zur Ermöglichung Entspannungsverdampfung der kosmetischen Zubereitung a), zu öffnen. Dies kann beispielsweise durch ein Bauteil zur Durchflussregelung b2, insbesondere ein Ventil, gewährleistet werden.

Der Behälter b1), in welchem die kosmetische Zubereitung erhitzt wird, steht vorzugsweise in Kontakt mit einem weiteren Behälter, aus welchem die zur Entspannungsverdampfung vorgesehene Menge der kosmetischen Zubereitung vor der Erhitzung in den Behälter b1) überführt wird. Der Zugang zwischen diesem Vorratsbehälter und Behälter b1) ist dabei über eine entsprechende Vorrichtung, beispielsweise ein Ventil, zu öffnen und zu schließen. Dieser weitere Behälter ist vorzugsweise in Form eines Vorratsbehälters ausgestaltet, das heißt, er umfasst bevorzugt ein Vielfaches, beispielsweise mehr als das Zehnfache, vorzugsweise mehr als das Fünfzigfache, der für einen Verdampfungsvorgang notwendigen Menge der kosmetischen Zubereitung. Mit anderen Worten weist der weitere Behälter/Vorratsbehälter vorzugsweise ein Vielfaches, beispielsweise mehr als das Zehnfache Volumen, vorzugsweise mehr als das Zwanzigfache und insbesondere mehr als das Fünfzigfache Volumen des Behälters b1) auf.

Ein weiterer besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 65 bis 75 Gew.-% polares Lösungsmittel;
   a2) 0,003 bis 3,0 Gew.-% direktziehenden Farbstoff;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht eine in dem geschlossenen Behälter b1) befindliche kosmetische Zubereitung zu erhitzen
   - eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Zwanzigfache und insbesondere mindestens das Fünfzigfache Volumen des Behälters b1) aufweist.

Der Vorratsbehälter ist kein Druckbehälter und die in dem Vorratsbehälter befindliche kosmetische Zusammensetzung befindet sich nicht unter Druck, mit anderen Worten entspricht der Druck im Inneren des Vorratsbehälters dem Umgebungsdruck (auch Luftdruck oder Atmosphärendruck). So umfassen entsprechende kosmetische Produkte beispielsweise keine Treibmittel. Auch verfügt das kosmetische Produkt über keine Pumpvorrichtung, die geeignet ist, die kosmetische Zubereitung ohne die Einwirkung der Vorrichtung zur Entspannungsverdampfung in die Umgebung freizusetzen oder zu versprühen.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 65 bis 75 Gew.-% polares Lösungsmittel;
   a2) 0,003 bis 3,0 Gew.-% direktziehenden Farbstoff;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht eine in dem geschlossenen Behälter b1) befindliche kosmetische Zubereitung zu erhitzen
   - eine Düse b4), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 65 bis 75 Gew.-% polares Lösungsmittel;
   a2) 0,003 bis 3,0 Gew.-% direktziehenden Farbstoff;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht eine in dem geschlossenen Behälter b1) befindliche kosmetische Zubereitung zu erhitzen
   - eine Düse b4), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst.

Bevorzugt werden weiterhin kosmetische Produkte, umfassend
a) eine kosmetische Zubereitung, enthaltend bezogen auf ihr Gesamtgewicht
   a1) 65 bis 75 Gew.-% polares Lösungsmittel;
   a2) 0,003 bis 3,0 Gew.-% direktziehenden Farbstoff;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht eine in dem geschlossenen Behälter b1) befindliche kosmetische Zubereitung zu erhitzen
   - eine Düse b4), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Zusammenfassend ist ein besonders bevorzugter Gegenstand der vorliegenden Erfindung daher ein kosmetisches Produkt, umfassend
a) eine kosmetische Zubereitung, enthaltend bezogen auf ihr Gesamtgewicht
   a1) 65 bis 75 Gew.-% polares Lösungsmittel;
   a2) 0,003 bis 3,0 Gew.-% direktziehenden Farbstoff;
b) eine Vorrichtung zur Entspannungsverdampfung der kosmetischen Zubereitung a), umfassend
   - einen mittels eines Ventils zu verschließenden und zu öffnenden Behälter b1)
   - eine Heizvorrichtung, welche es ermöglicht eine in dem geschlossenen Behälter b1) befindliche kosmetische Zubereitung zu erhitzen
   - eine Düse b4), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Neben den beiden zuvor beschriebenen Bestandteilen a1) und a2)) können die erfindungsgemäßen kosmetischen Zubereitungen a) weitere Wirk- oder Hilfsstoffe enthalten, wobei insbesondere solche Wirk- oder Hilfsstoffe bevorzugt werden, welche die Herstellbarkeit, Applizierbarkeit und/oder kosmetische Wirkung erfindungsgemäßer kosmetischer Zubereitungen verbessern.

Ein erster optionaler Bestandteil erfindungsgemäßer kosmetischer Zubereitungen sind die kationischen Polymere a3). Für die Herstellbarkeit und kosmetischer Wirkung der kosmetischen Zubereitung a) hat es sich als vorteilhaft erwiesen, wenn die kosmetische Zubereitung a), bezogen auf ihr Gesamtgewicht 0,1 bis 5,0 Gew.%, vorzugsweise 0,2 bis 3,0 Gew.-% und insbesondere 0,5 bis 2,0 Gew.-% kationisches Polymer a3) enthält.

Die Gruppe der kationischen Polymere a3) umfasst insbesondere die kationische Polymere mit den INCI Bezeichnungen Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polyquaternium-68 und Polyquaternium-69.

Eine zweite Gruppe optionaler Bestandteile der kosmetischen Zubereitungen a) bilden die zwitterionischen Tenside a4), deren Gewichtsanteil am Gesamtgewicht der kosmetischen Zubereitung vorzugsweise 0,1 bis 7,0 Gew.-%, bevorzugt 0,2 bis 5,0 Gew.-% und insbesondere 0,5 bis 3,0 Gew.-% beträgt.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder-SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat. Kosmetische Produkte, dadurch gekennzeichnet, dass das zwitterionische Tensid a4) aus der Gruppe der Substanzen mit der INCI-Bezeichung Amidopropylbetain, vorzugsweise aus der Gruppe der Substanzen mit der INCI-Bezeichnung Cocoamidopropylbetain ausgewählt ist, sind aufgrund ihrer vorteilhaften Eigenschaften besonders bevorzugt.

Eine dritte Gruppe bevorzugter optionaler Bestandteile der kosmetischen Zubereitung bilden die nichtionischen Tenside. Der Gewichtsanteil der nichtionischen Tenside am Gesamtgewicht der kosmetischen Zubereitung beträgt vorzugsweise 0,5 bis 3,0 Gew.-%.

Als bevorzugte nichtionische Tenside haben sich Alkylenoxid-Anlagerungsprodukte an gesättigte lineare Fettalkohole, Fettsäureester und Fettsäuren mit jeweils 2 bis 80 Mol Ethylenoxid und/oder 1 bis 5 Mol Propylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Dabei hat es sich als besonders vorteilhaft erwiesen, wenn das weitere nichtionische Tensid einen HLB-Wert oberhalb von 10, vorzugsweise oberhalb von 14 aufweist. Dazu ist notwendig, dass das nichtionische Tensid einen ausreichend hohen Ethoxylierungsgrad aufweist.

Eine weitere Ausführungsform des ersten Erfindungsgegenstands ist daher dadurch gekennzeichnet, dass das die kosmetische Zubereitung a) als nichtionisches Tensid mindestens ein ethoxyliertes Tensid mit mindestens 30 Ethylenoxideinheiten enthält.

Neben den entsprechend ethoxylierten Fettalkoholen sind erfindungsgemäß insbesondere die Anlagerungsprodukte von 30 bis 60 Mol Ethylenoxid an Rizinusöl und gehärtetes Rizinusöl besonders geeignet. Beispiele für solche geeigneten Tenside tragen die INCI-Bezeichnungen Steareth-30. Ceteareth- 30, Oleth-30, Ceteareth-50 oder PEG-40 Hydrogenated Castor Oil und PEG-60 Hydrogenated Castor Oil.

Das oder die zuvor genannten ethoxylierte(n) nichtionische(n) Tensid(e) kann (können) in einer bevorzugten Ausführungsform auch als Mischung mit Fettalkoholen und/oder ionischen Tensiden in der Oxidationsmittelzubereitung B eingesetzt werden.

Bevorzugte Beispiele für solche Mischungen sind beispielsweise Mischungen von Fettalkoholen, ethoxylierten nichtionischen Tensiden, insbesondere ethoxylierten gehärteten Rizinusölen und anionischen Sulfattensiden. Ein solches Handelsprodukt wird beispielsweise von der Firma BASF unter der Bezeichnung Emulgade F angeboten (INCI-Bezeichnung: Cetearyl Alcohol, PEG-40 Castor Oil, Sodium Cetearyl Sulfate).

Kosmetisches Produkte, dadurch gekennzeichnet, dass das nichtionische Tensid a5) aus der Gruppe der alkoxylierten Fettalkohole, vorzugsweise aus der Gruppe der ethoxylierten Fettalkohole ausgewählt ist, sind erfindungsgemäß bevorzugt.

Ein vierter bevorzugter Bestandteil der kosmetischen Zubereitungen a) sind die anionischen Tenside a6). Im Hinblick auf die Applizierbarkeit und kosmetische Wirkung ist es bevorzugt, wenn die kosmetische Zubereitung a), bezogen auf ihr Gesamtgewicht, 0,5 bis 3,0 Gew.-% anionisches Tensid a6) enthält.

Anionische Tenside im Sinne der Erfindung sind alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren, insbesondere der Formel RO(CH₂CH₂O)ₓCH₂COOH, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und -dialkylester sowie Sulfobernsteinsäuremono-alkylpolyoxyethylester; lineare Alkansulfonate; lineare α,-Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate. Besonders bevorzugt

Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Besonders bevorzugt sind C₈-C₂₀-Alkylsulfate, insbesondere Natriumcetearylsulfat und Natriumlaurylsulfat, sowie C₈-C₂₀-Alkylethersulfate mit 2 bis 12, vorzugsweise 2 bis 4 Ethylenoxidgruppen, insbesondere Natriumlaurylethersulfat (INCI: Sodium Laureth Sulfate).

Zur fünften Gruppe besonders bevorzugter optionaler Bestandteile der kosmetischen Zubereitung a) zählen die Polyalkylenglycole a7), insbesondere die Polyethylenglycole, deren Gewichtsanteil am Gesamtgewicht der kosmetischen Zubereitung vorzugsweise 1,0 bis 6,0 Gew.-% beträgt.

Erfindungsgemäß geeignet sind flüssige wie feste Polyethylenglykole (PEG), beispielsweise Polyalkylenglykole mit Molmassen von 250 (PEG-4) bis 3350 (PEG-75). Besonders bevorzugt werden Polyethylenglycole mit der INCI-Bezeichnung PEG-6, PEG-7, PEG-8, PEG-9, PEG-10, PEG-12, PEG-14, PEG-16, PEG-18, PEG-20, PEG-32 und PEG-40. Insbesondere bevorzugt sind die Polyethylenglycole mit der INCI-Bezeichnung PEG-8.

Eine letzte Gruppe besonders bevorzugter Bestandteile der kosmetischen Zubereitung a) bilden die polymeren Verdicker a8). Bevorzugte Verdicker sind ausgewählt aus der Gruppe der polymeren organischen Verdicker. Die polymeren organischen Verdicker können vernetzt oder unvernetzt sein.

In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil des polymeren Verdickers a8) am Gesamtgewicht der kosmetischen Zubereitung a) 0,5 bis 2,0 Gew.-% beträgt.

Beispiele für gebräuchliche Verdicker a8) sind polymere Verdickungsmittel mit den INCI-Bezeichnungen Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Yeast Beta-Glucan, Yeast Polysaccharides, Zea Mays (Corn) Starch.

Besonders bevorzugt sind polymere Verdicker a8) aus der Gruppe der Acrylsäure Homo- und Copolymere, vorzugsweise aus der Gruppe der Acrylsäure Homopolymere.

Neben den zuvor beschriebenen Inhaltsstoffen a1), a2) und a3) bis a8) können die erfindungsgemäßen kosmetischen Mittel weitere Wirk-, Hilfs- und Pflegestoffe enthalten.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

| | Formel 1 | Formel 2 | Formel 3 | Formel 4 | Formel 5 |
|---|---|---|---|---|---|
| polares Lösungsmittel a1) | 55 bis 85 | 60 bis 80 | 65 bis 75 | 73 | 70 |
| direktziehender Farbstoff a2) | 0,01 bis 10 | 0,02 bis 5,0 | 0,03 bis 3,0 | 1,2 | 2,1 |
| optionale Additive | add 100 | add 100 | add 100 | add 100 | add 100 |

| | Formel 6 | Formel 7 | Formel 8 | Formel 9 | Formel 10 |
|---|---|---|---|---|---|
| polares Lösungsmittel a1) | 55 bis 85 | 60 bis 80 | 65 bis 75 | 73 | 70 |
| anionischer direktziehender Farbstoff a2) | 0,01 bis 10 | 0,02 bis 5,0 | 0,03 bis 3,0 | 1,2 | 2,1 |
| optionale Additive | add 100 | add 100 | add 100 | add 100 | add 100 |

| | Formel 11 | Formel 12 | Formel 13 | Formel 14 | Formel 15 |
|---|---|---|---|---|---|
| polares Lösungsmittel a1) | 55 bis 85 | 60 bis 80 | 65 bis 75 | 73 | 70 |
| kationischer direktziehender Farbstoff a2) | 0,01 bis 10 | 0,02 bis 5,0 | 0,03 bis 3,0 | 1,2 | 2,1 |
| optionale Additive | add 100 | add 100 | add 100 | add 100 | add 100 |

| | Formel 16 | Formel 17 | Formel 18 | Formel 19 | Formel 20 |
|---|---|---|---|---|---|
| polares Lösungsmittel a1) | 55 bis 85 | 60 bis 80 | 65 bis 75 | 73 | 70 |
| nichtionischer direktziehender Farbstoff a2) | 0,01 bis 10 | 0,02 bis 5,0 | 0,03 bis 3,0 | 1,2 | 2,1 |
| optionale Additive | add 100 | add 100 | add 100 | add 100 | add 100 |

| | Formel 21 | Formel 22 | Formel 23 | Formel 24 | Formel 25 |
|---|---|---|---|---|---|
| polares Lösungsmittel a1) | 55 bis 85 | 60 bis 80 | 65 bis 75 | 73 | 70 |
| direktziehender Farbstoff a2) | 0,01 bis 10 | 0,02 bis 5,0 | 0,03 bis 3,0 | 1,2 | 2,1 |
| kationisches Polymer a3) | 0,1 bis 5,0 | 0,2 bis 3,0 | 0,5 bis 2,0 | 1,0 | 1,4 |
| optionale Additive | add 100 | add 100 | add 100 | add 100 | add 100 |

| | Formel 26 | Formel 27 | Formel 28 | Formel 29 | Formel 30 |
|---|---|---|---|---|---|
| polares Lösungsmittel a1) | 55 bis 85 | 60 bis 80 | 65 bis 75 | 73 | 70 |
| anionischer direktziehender Farbstoff a2) | 0,01 bis 10 | 0,02 bis 5,0 | 0,03 bis 3,0 | 1,2 | 2,1 |
| kationisches Polymer a3) | 0,1 bis 5,0 | 0,2 bis 3,0 | 0,5 bis 2,0 | 1,0 | 1,4 |
| optionale Additive | add 100 | add 100 | add 100 | add 100 | add 100 |

| | Formel 31 | Formel 32 | Formel 33 | Formel 34 | Formel 35 |
|---|---|---|---|---|---|
| polares Lösungsmittel a1) | 55 bis 85 | 60 bis 80 | 65 bis 75 | 73 | 70 |
| kationischer direktziehender Farbstoff a2) | 0,01 bis 10 | 0,02 bis 5,0 | 0,03 bis 3,0 | 1,2 | 2,1 |
| kationisches Polymer a3) | 0,1 bis 5,0 | 0,2 bis 3,0 | 0,5 bis 2,0 | 1,0 | 1,4 |
| optionale Additive | add 100 | add 100 | add 100 | add 100 | add 100 |

| | Formel 36 | Formel 37 | Formel 38 | Formel 39 | Formel 40 |
|---|---|---|---|---|---|
| polares Lösungsmittel a1) | 55 bis 85 | 60 bis 80 | 65 bis 75 | 73 | 70 |
| nichtionischer direktziehender Farbstoff a2) | 0,01 bis 10 | 0,02 bis 5,0 | 0,03 bis 3,0 | 1,2 | 2,1 |
| kationisches Polymer a3) | 0,1 bis 5,0 | 0,2 bis 3,0 | 0,5 bis 2,0 | 1,0 | 1,4 |
| optionale Additive | add 100 | add 100 | add 100 | add 100 | add 100 |

| | Formel 41 | Formel 42 | Formel 43 | Formel 44 | Formel 45 |
|---|---|---|---|---|---|
| polares Lösungsmittel a1) | 55 bis 85 | 60 bis 80 | 65 bis 75 | 73 | 70 |
| direktziehender Farbstoff a2) | 0,01 bis 10 | 0,02 bis 5,0 | 0,03 bis 3,0 | 1,2 | 2,1 |
| zwitterionisches Tensid a4) | 0,1 bis 7,0 | 0,2 bis 5,0 | 0,5 bis 3,0 | 1,8 | 1,2 |
| optionale Additive | add 100 | add 100 | add 100 | add 100 | add 100 |

| | Formel 46 | Formel 47 | Formel 48 | Formel 49 | Formel 50 |
|---|---|---|---|---|---|
| polares Lösungsmittel a1) | 55 bis 85 | 60 bis 80 | 65 bis 75 | 73 | 70 |
| anionischer direktziehender Farbstoff a2) | 0,01 bis 10 | 0,02 bis 5,0 | 0,03 bis 3,0 | 1,2 | 2,1 |
| zwitterionisches Tensid a4) | 0,1 bis 7,0 | 0,2 bis 5,0 | 0,5 bis 3,0 | 1,8 | 1,2 |
| optionale Additive | add 100 | add 100 | add 100 | add 100 | add 100 |

| | Formel 51 | Formel 52 | Formel 53 | Formel 54 | Formel 55 |
|---|---|---|---|---|---|
| polares Lösungsmittel a1) | 55 bis 85 | 60 bis 80 | 65 bis 75 | 73 | 70 |
| kationischer direktziehender Farbstoff a2) | 0,01 bis 10 | 0,02 bis 5,0 | 0,03 bis 3,0 | 1,2 | 2,1 |
| zwitterionisches Tensid a4) | 0,1 bis 7,0 | 0,2 bis 5,0 | 0,5 bis 3,0 | 1,8 | 1,2 |
| optionale Additive | add 100 | add 100 | add 100 | add 100 | add 100 |

| | Formel 56 | Formel 57 | Formel 58 | Formel 59 | Formel 60 |
|---|---|---|---|---|---|
| polares Lösungsmittel a1) | 55 bis 85 | 60 bis 80 | 65 bis 75 | 73 | 70 |
| nichtionischer direktziehender Farbstoff a2) | 0,01 bis 10 | 0,02 bis 5,0 | 0,03 bis 3,0 | 1,2 | 2,1 |
| zwitterionisches Tensid a4) | 0,1 bis 7,0 | 0,2 bis 5,0 | 0,5 bis 3,0 | 1,8 | 1,2 |
| optionale Additive | add 100 | add 100 | add 100 | add 100 | add 100 |

| | Formel 61 | Formel 62 | Formel 63 | Formel 64 | Formel 65 |
|---|---|---|---|---|---|
| polares Lösungsmittel a1) | 55 bis 85 | 60 bis 80 | 65 bis 75 | 73 | 70 |
| direktziehender Farbstoff a2) | 0,01 bis 10 | 0,02 bis 5,0 | 0,03 bis 3,0 | 1,2 | 2,1 |
| nichtionisches Tensid a5) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 66 | Formel 67 | Formel 68 | Formel 69 | Formel 70 |
|---|---|---|---|---|---|
| polares Lösungsmittel a1) | 55 bis 85 | 60 bis 80 | 65 bis 75 | 73 | 70 |
| anionischer direktziehender Farbstoff a2) | 0,01 bis 10 | 0,02 bis 5,0 | 0,03 bis 3,0 | 1,2 | 2,1 |
| nichtionisches Tensid a5) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 71 | Formel 72 | Formel 73 | Formel 74 | Formel 75 |
|---|---|---|---|---|---|
| polares Lösungsmittel a1) | 55 bis 85 | 60 bis 80 | 65 bis 75 | 73 | 70 |
| kationischer direktziehender Farbstoff a2) | 0,01 bis 10 | 0,02 bis 5,0 | 0,03 bis 3,0 | 1,2 | 2,1 |
| nichtionisches Tensid a5) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 76 | Formel 77 | Formel 78 | Formel 79 | Formel 80 |
|---|---|---|---|---|---|
| polares Lösungsmittel a1) | 55 bis 85 | 60 bis 80 | 65 bis 75 | 73 | 70 |
| nichtionischer direktziehender Farbstoff a2) | 0,01 bis 10 | 0,02 bis 5,0 | 0,03 bis 3,0 | 1,2 | 2,1 |
| nichtionisches Tensid a5) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 81 | Formel 82 | Formel 83 | Formel 84 | Formel 85 |
|---|---|---|---|---|---|
| polares Lösungsmittel a1) | 55 bis 85 | 60 bis 80 | 65 bis 75 | 73 | 70 |
| direktziehender Farbstoff a2) | 0,01 bis 10 | 0,02 bis 5,0 | 0,03 bis 3,0 | 1,2 | 2,1 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 86 | Formel 87 | Formel 88 | Formel 89 | Formel 90 |
|---|---|---|---|---|---|
| polares Lösungsmittel a1) | 55 bis 85 | 60 bis 80 | 65 bis 75 | 73 | 70 |
| anionischer direktziehender Farbstoff a2) | 0,01 bis 10 | 0,02 bis 5,0 | 0,03 bis 3,0 | 1,2 | 2,1 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 91 | Formel 92 | Formel 93 | Formel 94 | Formel 95 |
|---|---|---|---|---|---|
| polares Lösungsmittel a1) | 55 bis 85 | 60 bis 80 | 65 bis 75 | 73 | 70 |
| kationischer direktziehender Farbstoff a2) | 0,01 bis 10 | 0,02 bis 5,0 | 0,03 bis 3,0 | 1,2 | 2,1 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 96 | Formel 97 | Formel 98 | Formel 99 | Formel 100 |
|---|---|---|---|---|---|
| polares Lösungsmittel a1) | 55 bis 85 | 60 bis 80 | 65 bis 75 | 73 | 70 |
| nichtionischer direktziehender Farbstoff a2) | 0,01 bis 10 | 0,02 bis 5,0 | 0,03 bis 3,0 | 1,2 | 2,1 |
| anionisches Tensid a6) | 0,1 bis 6,0 | 0,2 bis 4,0 | 0,5 bis 3,0 | 1,2 | 1,5 |
| optionale Additive | add 100 | add 100 | add 100 | add 100 | add 100 |

| | Formel 101 | Formel 102 | Formel 103 | Formel 104 | Formel 105 |
|---|---|---|---|---|---|
| polares Lösungsmittel a1) | 55 bis 85 | 60 bis 80 | 65 bis 75 | 73 | 70 |
| direktziehender Farbstoff a2) | 0,01 bis 10 | 0,02 bis 5,0 | 0,03 bis 3,0 | 1,2 | 2,1 |
| Polyalkylenglycol a7) | 0,1 bis 10 | 0,5 bis 8,0 | 1,0 bis 6,0 | 4,0 | 5,0 |
| optionale Additive | add 100 | add 100 | add 100 | add 100 | add 100 |

| | Formel 106 | Formel 107 | Formel 108 | Formel 109 | Formel 110 |
|---|---|---|---|---|---|
| polares Lösungsmittel a1) | 55 bis 85 | 60 bis 80 | 65 bis 75 | 73 | 70 |
| anionischer direktziehender Farbstoff a2) | 0,01 bis 10 | 0,02 bis 5,0 | 0,03 bis 3,0 | 1,2 | 2,1 |
| Polyalkylenglycol a7) | 0,1 bis 10 | 0,5 bis 8,0 | 1,0 bis 6,0 | 4,0 | 5,0 |
| optionale Additive | add 100 | add 100 | add 100 | add 100 | add 100 |

| | Formel 111 | Formel 112 | Formel 113 | Formel 114 | Formel 115 |
|---|---|---|---|---|---|
| polares Lösungsmittel a1) | 55 bis 85 | 60 bis 80 | 65 bis 75 | 73 | 70 |
| kationischer direktziehender Farbstoff a2) | 0,01 bis 10 | 0,02 bis 5,0 | 0,03 bis 3,0 | 1,2 | 2,1 |
| Polyalkylenglycol a7) | 0,1 bis 10 | 0,5 bis 8,0 | 1,0 bis 6,0 | 4,0 | 5,0 |
| optionale Additive | add 100 | add 100 | add 100 | add 100 | add 100 |

| | Formel 116 | Formel 117 | Formel 118 | Formel 119 | Formel 120 |
|---|---|---|---|---|---|
| polares Lösungsmittel a1) | 55 bis 85 | 60 bis 80 | 65 bis 75 | 73 | 70 |
| nichtionischer direktziehender Farbstoff a2) | 0,01 bis 10 | 0,02 bis 5,0 | 0,03 bis 3,0 | 1,2 | 2,1 |
| Polyalkylenglycol a7) | 0,1 bis 10 | 0,5 bis 8,0 | 1,0 bis 6,0 | 4,0 | 5,0 |
| optionale Additive | add 100 | add 100 | add 100 | add 100 | add 100 |

| | Formel 121 | Formel 122 | Formel 123 | Formel 124 | Formel 125 |
|---|---|---|---|---|---|
| polares Lösungsmittel a1) | 55 bis 85 | 60 bis 80 | 65 bis 75 | 73 | 70 |
| direktziehender Farbstoff a2) | 0,01 bis 10 | 0,02 bis 5,0 | 0,03 bis 3,0 | 1,2 | 2,1 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 7,0 | 0,2 bis 5,0 | 0,5 bis 3,0 | 1,8 | 1,2 |
| nichtionisches Tensid a5) | 0,1 bis 6,0 | 0,2 bis 4,0 | 0,5 bis 3,0 | 1,2 | 1,5 |
| anionisches Tensid a6) | 0,1 bis 6,0 | 0,2 bis 4,0 | 0,5 bis 3,0 | 1,2 | 1,5 |
| Polyalkylenglycol a7) | 0,1 bis 10 | 0,5 bis 8,0 | 1,0 bis 6,0 | 4,0 | 5,0 |
| Verdicker a8) | 0,1 bis 6,0 | 0,2 bis 4,0 | 0,5 bis 2,0 | 1,0 | 0,5 |
| optionale Additive | add 100 | add 100 | add 100 | add 100 | add 100 |

| | Formel 126 | Formel 127 | Formel 128 | Formel 129 | Formel 130 |
|---|---|---|---|---|---|
| polares Lösungsmittel a1) | 55 bis 85 | 60 bis 80 | 65 bis 75 | 73 | 70 |
| anionischer direktziehender Farbstoff a2) | 0,01 bis 10 | 0,02 bis 5,0 | 0,03 bis 3,0 | 1,2 | 2,1 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 7,0 | 0,2 bis 5,0 | 0,5 bis 3,0 | 1,8 | 1,2 |
| nichtionisches Tensid a5) | 0,1 bis 6,0 | 0,2 bis 4,0 | 0,5 bis 3,0 | 1,2 | 1,5 |
| anionisches Tensid a6) | 0,1 bis 6,0 | 0,2 bis 4,0 | 0,5 bis 3,0 | 1,2 | 1,5 |
| Polyalkylenglycol a7) | 0,1 bis 10 | 0,5 bis 8,0 | 1,0 bis 6,0 | 4,0 | 5,0 |
| Verdicker a8) | 0,1 bis 6,0 | 0,2 bis 4,0 | 0,5 bis 2,0 | 1,0 | 0,5 |
| optionale Additive | add 100 | add 100 | add 100 | add 100 | add 100 |

| | Formel 131 | Formel 132 | Formel 133 | Formel 134 | Formel 135 |
|---|---|---|---|---|---|
| polares Lösungsmittel a1) | 55 bis 85 | 60 bis 80 | 65 bis 75 | 73 | 70 |
| kationischer direktziehender Farbstoff a2) | 0,01 bis 10 | 0,02 bis 5,0 | 0,03 bis 3,0 | 1,2 | 2,1 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 7,0 | 0,2 bis 5,0 | 0,5 bis 3,0 | 1,8 | 1,2 |
| nichtionisches Tensid a5) | 0,1 bis 6,0 | 0,2 bis 4,0 | 0,5 bis 3,0 | 1,2 | 1,5 |
| anionisches Tensid a6) | 0,1 bis 6,0 | 0,2 bis 4,0 | 0,5 bis 3,0 | 1,2 | 1,5 |
| Polyalkylenglycol a7) | 0,1 bis 10 | 0,5 bis 8,0 | 1,0 bis 6,0 | 4,0 | 5,0 |
| Verdicker a8) | 0,1 bis 6,0 | 0,2 bis 4,0 | 0,5 bis 2,0 | 1,0 | 0,5 |
| optionale Additive | add 100 | add 100 | add 100 | add 100 | add 100 |

| | Formel 136 | Formel 137 | Formel 138 | Formel 139 | Formel 140 |
|---|---|---|---|---|---|
| polares Lösungsmittel a1) | 55 bis 85 | 60 bis 80 | 65 bis 75 | 73 | 70 |
| nichtionischer direktziehender Farbstoff a2) | 0,01 bis 10 | 0,02 bis 5,0 | 0,03 bis 3,0 | 1,2 | 2,1 |
| kationisches Polymer a3) | 0,1 bis 5,0 | 0,2 bis 3,0 | 0,5 bis 2,0 | 1,0 | 1,4 |
| zwitterionisches Tensid a4) | 0,1 bis 7,0 | 0,2 bis 5,0 | 0,5 bis 3,0 | 1,8 | 1,2 |
| nichtionisches Tensid a5) | 0,1 bis 6,0 | 0,2 bis 4,0 | 0,5 bis 3,0 | 1,2 | 1,5 |
| anionisches Tensid a6) | 0,1 bis 6,0 | 0,2 bis 4,0 | 0,5 bis 3,0 | 1,2 | 1,5 |
| Polyalkylenglycol a7) | 0,1 bis 10 | 0,5 bis 8,0 | 1,0 bis 6,0 | 4,0 | 5,0 |
| Verdicker a8) | 0,1 bis 6,0 | 0,2 bis 4,0 | 0,5 bis 2,0 | 1,0 | 0,5 |
| optionale Additive | add 100 | add 100 | add 100 | add 100 | add 100 |

| | Formel 141 | Formel 142 | Formel 143 | Formel 144 | Formel 145 |
|---|---|---|---|---|---|
| Wasser | 55 bis 85 | 60 bis 80 | 65 bis 75 | 73 | 70 |
| direktziehender Farbstoff a2) | 0,01 bis 10 | 0,02 bis 5,0 | 0,03 bis 3,0 | 1,2 | 2,1 |
| Polyquaternium-6 | 0,1 bis 5,0 | 0,2 bis 3,0 | 0,5 bis 2,0 | 1,0 | 1,4 |
| Cocoamidopropylbetain | 0,1 bis 7,0 | 0,2 bis 5,0 | 0,5 bis 3,0 | 1,8 | 1,2 |
| PEG-40 Castor Oil | 0,1 bis 6,0 | 0,2 bis 4,0 | 0,5 bis 3,0 | 1,2 | 1,5 |
| Natriumlaurylethersulfat | 0,1 bis 6,0 | 0,2 bis 4,0 | 0,5 bis 3,0 | 1,2 | 1,5 |
| PEG-8 | 0,1 bis 10 | 0,5 bis 8,0 | 1,0 bis 6,0 | 4,0 | 5,0 |
| Carbomer | 0,1 bis 6,0 | 0,2 bis 4,0 | 0,5 bis 2,0 | 1,0 | 0,5 |
| optionale Additive | add 100 | add 100 | add 100 | add 100 | add 100 |

| | Formel 146 | Formel 147 | Formel 148 | Formel 149 | Formel 150 |
|---|---|---|---|---|---|
| Wasser | 55 bis 85 | 60 bis 80 | 65 bis 75 | 73 | 70 |
| nichtionischer direktziehender Farbstoff a2) | 0,01 bis 10 | 0,02 bis 5,0 | 0,03 bis 3,0 | 1,2 | 2,1 |
| Polyquaternium-6 | 0,1 bis 5,0 | 0,2 bis 3,0 | 0,5 bis 2,0 | 1,0 | 1,4 |
| Cocoamidopropylbetain | 0,1 bis 7,0 | 0,2 bis 5,0 | 0,5 bis 3,0 | 1,8 | 1,2 |
| PEG-40 Castor Oil | 0,1 bis 6,0 | 0,2 bis 4,0 | 0,5 bis 3,0 | 1,2 | 1,5 |
| Natriumlaurylethersulfat | 0,1 bis 6,0 | 0,2 bis 4,0 | 0,5 bis 3,0 | 1,2 | 1,5 |
| PEG-8 | 0,1 bis 10 | 0,5 bis 8,0 | 1,0 bis 6,0 | 4,0 | 5,0 |
| Carbomer | 0,1 bis 6,0 | 0,2 bis 4,0 | 0,5 bis 2,0 | 1,0 | 0,5 |
| optionale Additive | add 100 | add 100 | add 100 | add 100 | add 100 |

Ganz besonders bevorzugte kosmetische Zubereitungen enthalten neben den zuvor beschriebenen Bestandteilen a1) bis a6) nur geringe Mengen weiterer Wirk- und Hilfsstoffe. Kosmetische Zubereitungen, dadurch gekennzeichnet, dass der Gewichtsanteil der Bestandteile a1), a2) sowie, falls vorhanden, der optionalen Bestandteilen a3) bis a8) am Gesamtgewicht der kosmetischen Zubereitung mindestens 86 Gew.-%, vorzugsweise mindestens 90 Gew.-% und insbesondere mindestens 94 Gew.-% beträgt, sind aufgrund ihrer einfachen Herstellbarkeit und guten kosmetischen Wirkung besonders bevorzugt.

Wie eingangs ausgeführt eignen sich die erfindungsgemäßen kosmetischen Zubereitungen a) in besonderer Weise für die Applikation mittels einer Vorrichtung zur Entspannungsverdampfung. Ein weiterer Gegenstand der vorliegenden Anmeldung ist daher die Verwendung einer kosmetischen Zubereitung a) enthaltend, bezogen auf ihr Gesamtgewicht,
a1) 65 bis 75 Gew.-% polares Lösungsmittel;
a2) 0,003 bis 3,0 Gew.-% direktziehenden Farbstoff;
als Prozessgut in einer Vorrichtung zur Entspannungsverdampfung.

Gegenstand der vorliegenden Anmeldung ist zudem die Verwendung eines erfindungsgemäßen Produktes zur Beaufschlagung keratinhaltiger Fasern, insbesondere menschlicher Haare, mit einer kosmetischen Zubereitung a) bzw. zur Farbveränderung keratinhaltiger Fasern, insbesondere menschlicher Haare.

Ein Verfahren zur Farbveränderung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 65 bis 75 Gew.-% polares Lösungsmittel;
a2) 0,003 bis 3,0 Gew.-% direktziehenden Farbstoff;
beaufschlagt werden, ist ein weiterer Gegenstand der vorliegenden Anmeldung. Die kosmetische Zubereitung a) wird mittels der Vorrichtung zur Entspannungsverdampfung vorzugsweise in einen Sprühnebel überführt, welcher nachfolgend die keratinhaltigen Fasern beaufschlagt.

Um eine ausreichende Sprühwirkung zu erzielen, wird die kosmetische Zubereitung a) vorzugsweise auf Temperaturen oberhalb des Siedepunktes des in der kosmetischen Zubereitung a) enthaltenen polaren Lösungsmittels oder Lösungsmittelgemisches erhitzt.

Handelt es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches), wird die kosmetische Zubereitung vorzugsweise auf Temperaturen oberhalb 100°C, vorzugsweise auf Temperaturen von 100°C und 240°C, besonders bevorzugt auf Temperaturen von 140°C bis 160°C erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Ein bevorzugter Anmeldungsgegenstand ist ein Verfahren zur Farbveränderung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 65 bis 75 Gew.-% polares Lösungsmittel;
a2) 0,003 bis 3,0 Gew.-% direktziehenden Farbstoff;
beaufschlagt werden, wobei
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung
   a) in einen Behälter b1) überführt wird;
      - nachfolgend der Zugang zwischen Vorratsbehälter und Behälter b1) durch ein Bauteil zur Durchflussregelung, mittels dessen der Durchfluss der kosmetischen Zubereitung a) aus dem Vorratsbehälter in den Behälter b1) unterbrochen werden kann, unterbrochen wird;
      - nachfolgend die in dem gegen die Umgebung abgeschlossenen Behälter b1) befindliche kosmetische Zubereitung a) mittels einer Heizvorrichtung erhitzt wird, so dass der Druck im Inneren des Behälters b1) auf Werte oberhalb des Umgebungsdrucks, vorzugsweise auf Werte zwischen 1,1 und 8 bar, insbesondere auf Werte zwischen 1,2 und 4 bar ansteigt;
      - nachfolgend der unter einem Druck oberhalb des Umgebungsdrucks stehende Behälter b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung aus dem Behälter b1) in die Umgebung unter Minderung des zum Zeitpunkt der Behälteröffnung in dem Behälter b1) herrschenden Drucks, entspannt wird.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind besonders bevorzugt.

Bezüglich weiterer bevorzugter Ausführungsformen der erfindunsgemäßen Verwendungen und des erfindungsgemäßen Verfahrens gilt mutatis mutandis das zu den erfindungsgemäßen kosmetischen Zubereitungen a) und zu der Vorrichtung zur Entspannungsverdampfung b) Gesagte.

## Patentansprüche

1. Kosmetisches Produkt, umfassend
a) eine kosmetische Zubereitung (a), enthaltend bezogen auf ihr Gesamtgewicht
a1) 65 bis 75 Gew.-% polares Lösungsmittel;
a2) 0,003 bis 3,0 Gew.-% direktziehenden Farbstoff, wobei als direktziehender Farbstoff alternativ ein kationischer Farbstoff, ein anionischer Farbstoff oder ein nichtionischer Farbstoff eingesetzt wird;
b) eine Vorrichtung zur Entspannungsverdampfung der kosmetischen Zubereitung (a) mit
b1) einem Behälter (b1), welcher einen geschlossenen Innenraum definiert, in dem die kosmetische Zubereitung (a) aufgenommen werden kann,
b2) einem Ventil oder einem vergleichbar wirkenden Schließelement, um den wenigstens anteilsweise mit der kosmetischen Zubereitung (a) befüllten Innenraum des Behälters (b1) zu verschließen und zu öffnen,
b3) einer Heizvorrichtung, um die in dem geschlossenen Innenraum des Behälters (b1) befindliche kosmetische Zubereitung (a) unter Druckerhöhung zu erhitzen, sowie die erhitzte kosmetische Zubereitung (a) aus dem Innenraum des Behälters (b1) unter Druckminderung in die Umgebung zu entspannen,
b4) einer Düse, welche eine Zerstäubung der aus dem Behälter (b1) entweichenden kosmetischen Zubereitung (a) ermöglicht.

2. Kosmetisches Produkt nach einem der vorherigen Ansprüche, **dadurch gekennzeichnet, dass** das polare Lösungsmittel a1) ausgewählt ist aus der Gruppe Wasser und Ethanol.

3. Kosmetisches Produkt nach einem der vorherigen Ansprüche, **dadurch gekennzeichnet, dass** die kosmetische Zubereitung (a) bezogen auf ihr Gesamtgewicht 0,1 bis 5,0 Gew.%, vorzugsweise 0,2 bis 3,0 Gew.-% und insbesondere 0,5 bis 2,0 Gew.-% kationisches Polymer a3) enthält.

4. Kosmetisches Produkt nach einem der vorherigen Ansprüche, **dadurch gekennzeichnet, dass** die kosmetische Zubereitung (a) bezogen auf ihr Gesamtgewicht 0,1 bis 7,0 Gew.-%, vorzugsweise 0,2 bis 5,0 Gew.-% und insbesondere 0,5 bis 3,0 Gew.-% zwitterionisches Tensid a4) enthält.

5. Verwendung einer kosmetischen Zubereitung (a) enthaltend, bezogen auf ihr Gesamtgewicht,
a) eine kosmetische Zubereitung (a), enthaltend bezogen auf ihr Gesamtgewicht
a1) 65 bis 75 Gew.-% polares Lösungsmittel;
a2) 0,003 bis 3,0 Gew.-% direktziehenden Farbstoff, wobei als direktziehender Farbstoff alternativ ein kationischer Farbstoff, ein anionischer Farbstoff oder ein nichtionischer Farbstoff eingesetzt wird;
als Prozessgut in einer Vorrichtung zur Entspannungsverdampfung der kosmetischen Zubereitung (a) mit
b1) einem Behälter (b1), welcher einen geschlossenen Innenraum definiert, in dem die kosmetische Zubereitung (a) aufgenommen werden kann,
b2) einem Ventil oder einem vergleichbar wirkenden Schließelement, um den wenigstens anteilsweise mit der kosmetischen Zubereitung (a) befüllten Innenraum des Behälters (b1) zu verschließen und zu öffnen,
b3) einer Heizvorrichtung, um die in dem geschlossenen Innenraum des Behälters (b1) befindliche kosmetische Zubereitung (a) unter Druckerhöhung zu erhitzen, sowie die erhitzte kosmetische Zubereitung (a) aus dem Innenraum des Behälters (b1) unter Druckminderung in die Umgebung zu entspannen,
b4) einer Düse, welche eine Zerstäubung der aus dem Behälter (b1) entweichenden kosmetischen Zubereitung (a) ermöglicht.

6. Verwendung eines Produkts nach einem der Ansprüche 1 bis 4 zur Beaufschlagung keratinhaltiger Fasern, insbesondere menschlicher Haare, mit einer kosmetischen Zubereitung (a).

7. Verwendung eines Produkts nach einem der Ansprüche 1 bis 4 zur Farbveränderung keratinhaltiger Fasern, insbesondere menschlicher Haare.

8. Verfahren zur Farbveränderung keratinhaltiger Fasern, insbesondere menschlicher Haare, bei welchem die keratinhaltigen Fasern mittels einer Vorrichtung zur Entspannungsverdampfung einer kosmetischen Zubereitung (a) mit
b1) einem Behälter (b1), welcher einen geschlossenen Innenraum definiert, in dem die kosmetische Zubereitung (a) aufgenommen werden kann,
b2) einem Ventil oder einem vergleichbar wirkenden Schließelement, um den wenigstens anteilsweise mit der kosmetischen Zubereitung (a) befüllten Innenraum des Behälters (b1) zu verschließen und zu öffnen,
b3) einer Heizvorrichtung, um die in dem geschlossenen Innenraum des Behälters (b1) befindliche kosmetische Zubereitung (a) unter Druckerhöhung zu erhitzen, sowie die erhitzte kosmetische Zubereitung (a) aus dem Innenraum des Behälters (b1) unter Druckminderung in die Umgebung zu entspannen,
b4) einer Düse, welche eine Zerstäubung der aus dem Behälter (b1) entweichenden kosmetischen Zubereitung (a) ermöglicht,
mit einer kosmetischen Zubereitung (a) enthaltend bezogen auf ihr Gesamtgewicht
a1) 65 bis 75 Gew.-% polares Lösungsmittel;
a2) 0,003 bis 3,0 Gew.-% direktziehenden Farbstoff, wobei als direktziehender Farbstoff alternativ ein kationischer Farbstoff, ein anionischer Farbstoff oder ein nichtionischer Farbstoff eingesetzt wird;
beaufschlagt werden.

9. Verfahren nach Anspruch 8, **dadurch gekennzeichnet, dass**
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung (a) in einen Behälter (b1) überführt wird;
- nachfolgend der Zugang zwischen Vorratsbehälter und Behälter (b1) durch ein Bauteil zur Durchflussregelung, mittels dessen der Durchfluss der kosmetischen Zubereitung a) aus dem Vorratsbehälter in den Behälter (b1) unterbrochen werden kann, unterbrochen wird;
- nachfolgend die in dem gegen die Umgebung abgeschlossenen Behälter (b1) befindliche kosmetische Zubereitung (a) mittels einer Heizvorrichtung erhitzt wird, so dass der Druck im Inneren des Behälters (b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter (b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter (b1) befindlichen kosmetischen Zubereitung (a) aus dem Behälter (b1) in die Umgebung unter Minderung des zum Zeitpunkt der Behälteröffnung in dem Behälter (b1) herrschenden Drucks, entspannt wird.

## Claims

1. A cosmetic product, comprising
a) a cosmetic preparation (a) containing, based on the total weight thereof,
a1) 65 to 75 wt.% polar solvent;
a2) 0.003 to 3.0 wt.% direct dye, wherein alternatively a cationic dye, an anionic dye or a non-ionic dye is used as the direct dye;
b) a device for flash evaporation of the cosmetic preparation (a), having b1) a container (b1) which defines a closed interior in which the cosmetic preparation (a) can be received,
b2) a valve or a similarly functioning closure element for closing and opening the interior of the container (b1), which is at least partly filled with the cosmetic preparation (a),
b3) a heating device for heating, under increased pressure, the cosmetic preparation (a) that is located in the closed interior of the container (b1), and releasing, under reduced pressure, the heated cosmetic preparation (a) from the interior of the container (b1) into the surroundings,
b4) a nozzle which allows atomization of the cosmetic preparation (a) escaping from the container (b1).

2. The cosmetic product according to one of the preceding claims, **characterized in that** the polar solvent a1) is selected from the group consisting of water and ethanol.

3. The cosmetic product according to one of the preceding claims, **characterized in that** the cosmetic preparation (a) contains, based on the total weight thereof, 0.1 to 5.0 wt.%, preferably 0.2 to 3.0 wt.%, and in particular 0.5 to 2.0 wt.%, of a cationic polymer a3).

4. The cosmetic product according to one of the preceding claims, **characterized in that** the cosmetic preparation (a) contains, based on the total weight thereof, 0.1 to 7.0 wt.%, preferably 0.2 to 5.0 wt.%, and in particular 0.5 to 3.0 wt.%, of a zwitterionic surfactant a4).

5. The use of a cosmetic preparation (a) containing, based on the total weight thereof,
a) a cosmetic preparation (a) containing, based on the total weight thereof,
a1) 65 to 75 wt.% polar solvent;
a2) 0.003 to 3.0 wt.% direct dye, wherein alternatively a cationic dye, an anionic dye or a non-ionic dye is used as the direct dye;
as a process material in a device for flash evaporation of the cosmetic preparation (a), having
b1) a container (b1) which defines a closed interior in which the cosmetic preparation (a) can be received,
b2) a valve or a similarly functioning closure element for closing and opening the interior of the container (b1), which is at least partly filled with the cosmetic preparation (a),
b3) a heating device for heating, under increased pressure, the cosmetic preparation (a) that is located in the closed interior of the container (b1), and releasing, under reduced pressure, the heated cosmetic preparation (a) from the interior of the container (b1) into the surroundings,
b4) a nozzle which allows atomization of the cosmetic preparation (a) escaping from the container (b1).

6. The use of a product according to one of claims 1 to 4 for applying a cosmetic preparation (a) to keratin-containing fibers, in particular human hair.

7. The use of a product according to one of claims 1 to 4 for changing the color of keratin-containing fibers, in particular human hair.

8. A method for treating keratin-containing fibers, in particular human hair, in which the keratin-containing fibers are acted upon by a device for flash evaporation of a cosmetic preparation (a), having
b1) a container (b1) which defines a closed interior in which the cosmetic preparation (a) can be received,
b2) a valve or a similarly functioning closure element for closing and opening the interior of the container (b1), which is at least partly filled with the cosmetic preparation (a),
b3) a heating device for heating, under increased pressure, the cosmetic preparation (a) that is located in the closed interior of the container (b1), and releasing, under reduced pressure, the heated cosmetic preparation (a) from the interior of the container (b1) into the surroundings,
b4) a nozzle which allows atomization of the cosmetic preparation (a) escaping from the container (b1),
with a cosmetic preparation (a) containing, based on the total weight thereof,
a1) 65 to 75 wt.% polar solvent;
a2) 0.003 to 3.0 wt.% direct dye, wherein alternatively a cationic dye, an anionic dye or a non-ionic dye is used as the direct dye.

9. The method according to claim 8, **characterized in that**
- some of the cosmetic preparation (a) located in a reservoir, in the interior of which a pressure prevails that corresponds to the ambient pressure, is transferred from said reservoir into a container (b1);
- access between the reservoir and the container (b1) is subsequently interrupted by a component for flow control, by means of which the flow of the cosmetic preparation a) from the reservoir into the container (b1) can be interrupted;
- the cosmetic preparation (a) located in the container (b1) closed off from the surroundings is subsequently heated by means of a heating device, so that the pressure in the interior of the container (b1) rises to values greater than the ambient pressure, preferably to values between 1.1 and 8 bar, in particular to values between 1.2 and 4 bar;
- the container (b1) which is pressurized above the ambient pressure is subsequently opened in such a manner that the discharge of at least some, preferably at least 50 wt.%, more preferably at least 80 wt.% and in particular at least 90 wt.%, of the cosmetic preparation (a) located in the container (b1) is released from the container (b1) into the surroundings by the pressure prevailing in the container (b1) at the time at which the container is opened being reduced.

## Revendications

1. Produit cosmétique, comprenant
a) une préparation cosmétique (a) contenant, par rapport à son poids total,
a1) 65 à 75 % en poids de solvant polaire ;
a2) 0,003 à 3,0 % en poids de colorant à action directe, dans lequel, en guise de colorant à action directe, en variante, un colorant cationique, un colorant anionique ou un colorant non ionique est utilisé ;
b) un dispositif permettant la vaporisation par détente de la préparation cosmétique (a) comportant b1) un récipient (b1) qui définit un espace intérieur fermé dans lequel la préparation cosmétique (a) peut être reçue,
b2) une soupape ou un élément de fermeture à action comparable afin de fermer et d'ouvrir l'espace intérieur du récipient (b1) rempli au moins partiellement de la préparation cosmétique (a),
b3) un dispositif de chauffage afin de chauffer la préparation cosmétique (a) se trouvant dans l'espace intérieur fermé du récipient (b1) par augmentation de la pression, ainsi que de vaporiser par détente la préparation cosmétique (a) chauffée hors de l'espace intérieur du récipient (b1) et dans l'environnement par diminution de la pression,
b4) une buse permettant une atomisation de la préparation cosmétique (a) s'échappant du récipient (b1).

2. Produit cosmétique selon l'une des revendications précédentes, **caractérisé en ce que** le solvant polaire a1) est choisi dans le groupe constitué d'eau et d'éthanol.

3. Produit cosmétique selon l'une des revendications précédentes, **caractérisé en ce que** la préparation cosmétique (a) contient, par rapport à son poids total, 0,1 à 5,0 % en poids, de préférence 0,2 à 3,0 % en poids et en particulier 0,5 à 2,0 % en poids d'un polymère cationique a3).

4. Produit cosmétique selon l'une des revendications précédentes, **caractérisé en ce que** la préparation cosmétique (a) contient, par rapport à son poids total, 0,1 à 7,0 % en poids, de préférence 0,2 à 5,0 % en poids et en particulier 0,5 à 3,0 % en poids d'un tensioactif zwitterionique a4).

5. Utilisation d'une préparation cosmétique (a) contenant, par rapport à son poids total,
a) une préparation cosmétique (a) contenant, par rapport à son poids total,
a1) 65 à 75 % en poids de solvant polaire ;
a2) 0,003 à 3,0 % en poids de colorant à action directe, dans lequel, en guise de colorant à action directe, en variante, un colorant cationique, un colorant anionique ou un colorant non ionique est utilisé ;
en tant que matériau de processus dans un dispositif permettant la vaporisation par détente de la préparation cosmétique (a) comportant
b1) un récipient (b1) qui définit un espace intérieur fermé dans lequel la préparation cosmétique (a) peut être reçue,
b2) une soupape ou un élément de fermeture à action comparable afin de fermer et d'ouvrir l'espace intérieur du récipient (b1) rempli au moins partiellement de la préparation cosmétique (a),
b3) un dispositif de chauffage afin de chauffer la préparation cosmétique (a) se trouvant dans l'espace intérieur fermé du récipient (b1) par augmentation de la pression, ainsi que de vaporiser par détente la préparation cosmétique (a) chauffée hors de l'espace intérieur du récipient (b1) et dans l'environnement par diminution de la pression,
b4) une buse permettant une atomisation de la préparation cosmétique (a) s'échappant du récipient (b1).

6. Utilisation d'un produit selon l'une des revendications 1 à 4 permettant l'application d'une préparation cosmétique (a) sur des fibres kératiniques, en particulier sur des cheveux humains.

7. Utilisation d'un produit selon l'une des revendications 1 à 4 pour le changement de couleur de fibres kératiniques, en particulier de cheveux humains.

8. Procédé permettant le changement de couleur de fibres kératiniques, en particulier de cheveux humains, dans lequel est appliquée sur les fibres kératiniques, au moyen d'un dispositif permettant la vaporisation par détente d'une préparation cosmétique (a) comportant
b1) un récipient (b1) qui définit un espace intérieur fermé dans lequel la préparation cosmétique (a) peut être reçue,
b2) une soupape ou un élément de fermeture à action comparable afin de fermer et d'ouvrir l'espace intérieur du récipient (b1) rempli au moins partiellement de la préparation cosmétique (a),
b3) un dispositif de chauffage afin de chauffer la préparation cosmétique (a) se trouvant dans l'espace intérieur fermé du récipient (b1) par augmentation de la pression, ainsi que de vaporiser par détente la préparation cosmétique (a) chauffée hors de l'espace intérieur du récipient (b1) et dans l'environnement par diminution de la pression,
b4) une buse permettant une atomisation de la préparation cosmétique (a) s'échappant du récipient (b1),
une préparation cosmétique (a) contenant, par rapport à son poids total,
a1) 65 à 75 % en poids de solvant polaire ;
a2) 0,003 à 3,0 % en poids de colorant à action directe, dans lequel, en guise de colorant à action directe, en variante, un colorant cationique, un colorant anionique ou un colorant non ionique est utilisé.

9. Procédé selon la revendication 8, **caractérisé en ce que**
- à partir d'un réservoir de stockage à l'intérieur duquel règne une pression correspondant à la pression environnante, une fraction de la préparation cosmétique (a) se trouvant dans ledit réservoir de stockage est transférée dans un récipient (b1) ;
- l'accès entre le réservoir de stockage et le récipient (b1) est ensuite interrompu par un composant permettant la régulation du débit, au moyen duquel le débit de la préparation cosmétique a) hors du réservoir de stockage et dans le récipient (b1) peut être interrompu ;
- ensuite, la préparation cosmétique (a) se trouvant dans le récipient (b1) fermé par rapport à l'environnement est chauffée au moyen d'un dispositif de chauffage, de sorte que la pression à l'intérieur du récipient (b1) augmente jusqu'à des valeurs supérieures à la pression environnante, de préférence jusqu'à des valeurs comprises entre 1,1 et 8 bar, en particulier jusqu'à des valeurs comprises entre 1,2 et 4 bar ;
- le récipient (b1) se trouvant à une pression supérieure à la pression environnante est ensuite ouvert de manière à vaporiser par détente la sortie d'au moins une fraction, de préférence d'au moins 50 % en poids, de manière particulièrement préférée d'au moins 80 % en poids et en particulier d'au moins 90 % en poids de la préparation cosmétique (a) se trouvant dans le récipient (b1) hors du récipient (b1) et dans l'environnement, par diminution de la pression régnant dans le récipient (b1) au moment de l'ouverture du récipient.
